# EUROPÄISCHE PATENTSCHRIFT

(11) **EP 1 007 927 B1**
(45) Veröffentlichungstag und Bekanntmachung des Hinweises auf die Patenterteilung: **16.01.2002**
(21) Anmeldenummer: 97943886.8
(22) Anmeldetag: 23.09.1997
(51) Int. Cl.: G01L 5/24, A61B 5/00, A61B 5/0448, B25B 23/14, F16D 7/08, F16D 7/02, H02K 49/10

(54) **APPLIKATOR FÜR PULSOXIMETRIESENSOR MIT DREHMOMENTBEGRENZER**
APPLICATOR FOR PULSOXYMETRIC SENSOR WITH TORQUE LIMITER
APPLICATEUR A LIMITEUR DE COUPLE POUR CAPTEUR PULSOXYMETRIQUE

(30) Priorität: 26.09.1996 DE 19639648
(43) Veröffentlichungstag der Anmeldung: 14.06.2000
(73) Patentinhaber: Buschmann, Johannes, 81669 München (DE)
(72) Erfinder: Buschmann, Johannes, 81669 München (DE)
(86) Internationale Anmeldenummer: EP9705213
(87) Internationale Veröffentlichungsnummer: WO9813678

(56) Entgegenhaltungen:
- EP-A- 0 387 980
- WO-A-85/03754
- DE-A- 2 405 305
- DE-C- 3 710 769
- DE-C- 3 810 008
- US-A- 3 942 337
- US-A- 5 313 765
- PATENT ABSTRACTS OF JAPAN vol. 014, no. 311 (M-0994), 4.Juli 1990 & JP 02 102923 A (MITSUBA ELECTRIC MFG CO LTD), 16.April 1990,

## Beschreibung

Die Erfindung betrifft einen Applikator für in der Medizin anzuwendende Sensoren.

In der Medizin ist es bekannt, Sensoren, insbesondere Spiralsensoren, in die Haut des Patienten durch Drehung einzustechen, also einen Spiralsensor mit einem in der Regel sehr spitzen vorderen Ende durch eine spiralförmige Bewegung, bestehend aus Anpreßdruck und Drehmoment, in die Haut des Patienten einzuschrauben.

Dies geschieht beispielsweise bei den sehr weit verbreiteten Kopfschwartenelektroden, mit deren Hilfe nach Öffnung der Fruchtblase bei einem ungeborenen Kind ein Sensor zur Messung des EKG's des ungeborenen Kindes in dessen Kopfhaut verankert wird.

Auch optische Sensoren können in den Spitzen derartiger Spiralsensoren untergebracht sein, beispielsweise für die fetale Pulsoximetrie, mit deren Hilfe eine direkte Überwachung der Sauerstoffsättigung im Blut des ungeborenen Kindes überwacht werden kann.

Einen solchen Sensor offenbart die DE-C-3 810 008.

Da jedoch die Spiralsensoren an einer nicht einsehbaren Stelle, nämlich hinter der Scheide der Mutter in der Gebärmutter, angewendet werden muß, besteht für den Anwender das Problem, diese Spiralsensoren mit dem richtigen Drehmoment einzuschrauben. Bei zu stark angewandtem Drehmoment sind Verletzungen der Kopfschwarte möglich, aber auch eine Zerstörung des Sensors bzw. Applikators oder es kann durch zu hohen Axialdruck des Sensors auf den Patienten die Durchblutung im Anwendungsbereich eingeschränkt werden.

Bei zu geringem angewandtem Drehmoment sitzt der Sensor nur schlecht verankert in der Haut des Patienten, und er löst sich bei den während der Geburt auftretenden dynamischen Belastungen bzw. bei vaginalen Untersuchungen während der Geburt wieder aus dem Gewebe des Patienten. Darüber hinaus hat eine zu schwache Applikation Signalverfälschungen der vom Sensor gelieferten Signale zur Folge.

Das Aufbringen des richtigen Drehmomentes wird zusätzlich dadurch erschwert, daß sich der Sensor am vorderen Ende eines dünnen, langen Kunststoffstabes bzw. Kunststoffrohres befindet, und der Anwender dabei nur das hintere Handhabungsende dieses stabförmigen Applikators dreht. Bei einem ca. 20 cm langen, aber nur zwei oder drei Millimeter dicken Stab erschwert die Biegung und Torsion dieses stabförmigen Applikators ein Gefühl des Anwenders für das richtige Drehmoment.

Es ist daher die Aufgabe gemäß der vorliegenden Erfindung, einen Applikator zu schaffen, der das Drehmoment beim Einschrauben des Sensors in das Gewebe des Patienten wenigstens im Hinblick auf ein maximales Moment begrenzt, gleichzeitig aber auch das Einschrauben mit einem notwendigen minimalen Drehmoment sicherstellt.

Somit kann das durch den Drehmomentbegrenzer sichergestellte minimate Drehmoment mit dem maximalen Drehmoment zusammenfallen zu einem Schwellenwert, nämlich dem Schwellenwert, bei dem der Drehmomentbegrenzer durchrutscht.

Diese Aufgabe wird durch die Merkmale des Anspruchs 1 gelöst. Durch die Anordnung eines Drehmomentbegrenzers in bzw. am Applikator ist es sichergestellt, daß während des Einschraubens kein größeres Drehmoment auftreten kann als durch den Drehmomentbegrenzer vorgegeben.

Zusätzlich ist es wichtig, daß eine Wahrnehmbarkeit des Durchrutschens des Drehmomentbegrenzers gegeben ist, also eine Vorrichtung, die das Durchrutschen sehen oder wenigstens mit der ausübenden Hand spüren läßt.

Ein derartiger Drehmomentbegrenzer kann sowohl ein maximales als auch ein minimales Drehmoment vorgeben, um durch die Drehmomentbegrenzung nach unten in Richtung auf einen Minimalwert ein zu leichtes Applizieren und damit zu schlechtes Befestigen des Sensors im Gewebe des Patienten zu verhindern.

Eine besonders einfache Bauform eines Drehmomentbegrenzers stellt dabei eine Rutschkupplung dar, wobei es sich sowohl um eine mechanische, also kraftschlüssig oder formschlüssig wirkende, Rutschkupplung handeln kann als auch um eine magnetische, insbesondere elektromagnetische Rutschkupplung, wobei bei der elektromagnetischen Rutschkupplung durch elektrische Beeinflussung der Magnetwirkung die Ober- und Untergrenzen des Drehmomentes leicht einstellbar, leicht kontrollierbar und auch auf einfache Art und Weise schnell deaktiviert werden können.

Dabei scheint es auf den ersten Blick sinnvoll zu sein, den Drehmomentbegrenzer, beispielsweise die Rutschkupplung, möglichst nahe am Sensor, mithin also am vorderen, dem Sensorende, des Applikators, etwa zwischen dem Applikator und dem Sensor, vorzusehen, um den Einfluß des tordierbaren Applikators auf das auf den Patienten einwirkende Drehmoment auszuschließen.

Der Nachteil dieser Lösung liegt jedoch darin, daß sich dann der Drehmomentbegrenzer bei fetaler Anwendung innerhalb des Körpers der Mutter und damit unter Beeinflussung durch Körperflüssigkeiten und mechanischen Druck der umgebenden Vagina befindet, was die Funktion des Drehmomentbegrenzers unerwünscht beeinflussen kann.

Bei Anordnung des Applikators am hinteren Handhabungsende, welches außerhalb des Körpers der Mutter bei der Anwendung angeordnet ist, werden derartige störende Einflüsse durch Körperflüssigkeiten etc. vermieden. Um durch den zwischen Drehmomentbegrenzer und Sensor zwischengeordneten, tordierbaren Applikatorstab eine Beeinflussung auf das Drehmoment am Sensor zu vermeiden, muß das durch den Drehmomentbegrenzer maximal applizierbare Drehmoment niedriger liegen als das Drehmoment, durch welches der Applikatorstab zerstört werden kann. Eine leichte Torsion des Applikatorstabes ist dabei unschädlich, da diese Torsion lediglich einen Winkelversatz zwischen Handhabungsseite und Sensorseite des Applikatorstabes bewirkt, nicht jedoch die Höhe des auf den Patienten einwirkenden Drehmomentes, sofern dieses das durch den Drehmomentbegrenzer vorgegebene Maximaldrehmoment ist.

Ein derartiges fest vorgegebenes Drehmoment aufgrund der Drehmomentbegrenzung hat eine Vielzahl von Vorteilen:

Die an den Sensor angeschlossenen Auswerteeinrichtungen geben aussagefähige Werte ab, da sowohl die elektrische als auch die optische Ankopplung an das Gewebe zuverlässiger ist, der Einfluß auf die Gewebedurchblutung des Patienten bleibt minimai, seine Gewebsphysiologie unbeeinflußt und die Gewebstraumatisierung minimal. Auch die Verletzungsgefahr des Gewebes des Patienten wird minimiert, so daß dies alles auch ein juristisches Argument bei Auseinandersetzungen mit dem Patienten darstellt, da dieser Drehmomentbegrenzer die Anwendung eines zu hohen und ggf. auch eines zu geringen Drehmomentes ausschließt. Ferner wird die Zerstörung des Sensors durch Überbeanspruchung ebenso vermieden wie eine Notwendigkeit einer erneuten Applikation bei zu geringer Befestigung wegen zu geringen Anbring-Drehmomentes. Auch weniger geübte Anwender können sich von vornherein bei der Applikation sicher fühlen.

Bei der Unterbringung des Drehmomentbegrenzers am Handhabungsende des Applikators empfiehlt sich die Unterbringung insbesondere in einem Handgriffteil, welches lösbar mit dem Einwegapplikator verbunden werden kann, jedoch mehrfach wiederverwendbar ist.

Dabei kann der Drehmomentbegrenzer am Übergang zwischen dem Applikatorstab und dem Handhabungsteil untergebracht sein, oder zwischen zwei relativ zueinander bewegbaren Teilen des Handhabungsteiles, nämlich dem Applikatorflansch, an welchem das Handhabungsende des Applikatorstabes befestigbar ist und dem Handgriffteil, welches der Benutzer in der Hand hält.

Dabei ist es ebenfalls wichtig, daß das Handgriffteil einen Durchmesser aufweist, der etwa den Durchmesser der bisher üblichen, ohne Drehmomentbegrenzer ausgestatteten Applikatoren entspricht, um durch einen unterschiedlichen Greifdurchmesser nicht das subjektive Gefühl für das durch den Drehmomentbegrenzer vorgegebene Moment zu verfälschen, was einen Vertrauensschwund der Anwender in den Drehmomentbegrenzer zur Folge hätte.

Bei einer Anordnung des Drehmomentbegrenzers am vorderen Ende des Applikators, also z. B. zwischen Applikatorstab und Sensor, kann wegen der Einwegkonzeption des Applikators nur eine mechanisch einfache, leicht und billig herzustellende Bauform in Frage kommen. Dies ist beispielsweise die Ausbildung der Zusammenwirkung des vorderen Endes des Applikators und des Bauteiles, welches den Sensor unmittelbar trägt als mechanische Rutschkupplung, indem eines der Teile eine unrunde Außenkontur, z. B. eine elliptische oder vieleckige Außenkontur aufweist, und von dem anderen Bauteil radial umschlossen wird, welches eine analog geformte Innenkontur aufweist.

Wenn eines der Bauteile, insbesondere das umschließende Bauteil, eine relativ große Materialelastizität aufweist, und zwischen der Innen- und der Außenkontur ausreichend Spiel vorgesehen ist, wird das innere Teil bei einem maximal anzuwendenden Drehmoment gegenüber dem äußeren Teil durchrutschen, wodurch eine maximale Drehmomentbegrenzung gegeben ist. Nach dem Aufbringen dieses maximalen Drehmomentes wird der Applikatorstab wie üblich entfernt, so daß nur noch der Sensor und die vom Sensor weg führenden Signalleitungen am Patienten verbleiben.

Eine z. B. magnetische Rutschkupplung in dem Einwegteil, also zwischen Applikator und Sensor, anzuordnen, wäre zu kostenintensiv.

Eine derartige Lösung ist jedoch in Form eines vielfach wiederverwendbarem Handhabungsteiles, welches im Gegensatz zum Applikator selbst, der ja in der Regel aus Kunststoff besteht, aus stabilem Metall hergestellt werden kann, vertretbar. An dem Applikatorflansch des Handhabungsteiles ist das hintere Handhabungsende des Applikatorstabes mittels Einrasten, Befestigen mittels Klemmschraube etc. schnell und einfach befestigbar. Das Handhabungsteil besteht dabei aus zwei relativ zueinander bewegbaren Teilen, welche zwischen sich eine Rutschkupplung aufnehmen.

Diese Rutschkupplung kann die vorbeschriebene Variante oder eine aufwendigere mechanische Rutschkupplung mit federnden Rastteilen sein, oder eine magnetische oder elektromagnetische Rutschkupplung.

Bei einer magnetischen Rutschkupplung können die zusammenwirkenden Magnete an den gegeneinander gerichteten Flächen des Applikatorflansches bzw. Handgriffteiles des Handhabungsteiles angeordnet sein. Wenn dabei auf dem einen Teil alle Magnete so angeordnet sind, daß sie mit z. B. ihrem positiven Ende gegen das andere Teil ragen, und die Magnete am anderen Teil umgekehrt aufgebracht sind, also alle Magnetpaarungen sich gegenseitig anziehen, wird bei der Anwendung aufgrund der Anziehungskräfte der Magneten der Applikatorflansch und das Handgriffteil den minimal möglichen Abstand zueinander einnehmen, d. h., der Anwender, der zusätzlich in axialer Richtung schieben muß, bringt diesen Axialdruck unmittelbar am Gewebe des Patienten auf, ohne daß hierdurch zunächst ein Funktionsabstand zwischen den Teilen der Rutschkupplung verringert wird.

Bei Anordnung der Magnete in den gegeneinander gerichteten Stirnflächen muß ein definierter Abstand zwischen diesen Stirnflächen durch Anordnung eines Distanzringes, der beispielsweise aus Kunststoff besteht, vorgegeben werden, um die bei wechselndem Abstand stark variierenden Anziehungskräfte zwischen den Magneten, die ja das maximal erzielbare Drehmoment bedingen, bei Serienfertigung des Handhabungsteiles im Vergleich einzustellen.

Dieses Problem entfällt, wenn die Magnete im gegeneinander radial gerichteten Flächen des Applikatorflansches einerseits und des Handgriffteiles andererseits untergebracht sind, und diese Teile aneinander gelagert sind, und dadurch den immer gleichen radialen Abstand zueinander einnehmen.

Durch eine derartige magnetische Rutschkupplung wird eine maximale Drehmomentbegrenzung vorgenommen. Bei Ausbildung der Magnete als Elektromagnete kann das maximal aufzubringende Drehmoment eingestellt werden. Wenn bei einer derartigen magnetischen Lösung zusätzlich ein minimales Drehmoment vorgegeben werden soll, kann dies beispielsweise über die zu überwindende Reibung zwischen den beiden Teilen der Rutschkupplung vorgegeben werden, sofern während der Relativdrehung der Teile der Rutschkupplung zueinander kein magnetischer Einfluß gegeben ist. Dies ist beispielsweise dann möglich, wenn sich nur eine oder sehr wenige Magnetpaarungen über den Umfang der Rutschkupplung verteilt angeordnet sind, so daß im Bereich dazwischen keine magnetische Wechselwirkung auftritt, sondern nur die mechanische Reibung zwischen den beiden Teilen der Rutschkupplung. Durch axiale Vorspannung zwischen den beiden Teilen kann diese eine minimale Drehmomentbegrenzung bieten.

Eine Ausführungsform gemäß der Erfindung ist nachfolgend anhand der Figuren beispielhaft näher beschrieben. Es zeigen
- Fig. 1: einen Applikator mit magnetischer Rutschkupplung und
- Fig. 2: einen Applikator mit mechanischer Rutschkupplung,
- Fig. 3:: einen Längsschnitt durch eine mechanische Rutschkupplung, und
- Fig. 4 und 5:: Querschnitte durch die Rutschkupplung gemäß Fig. 3 entlang der Linien IV-IV bzw. V-V.

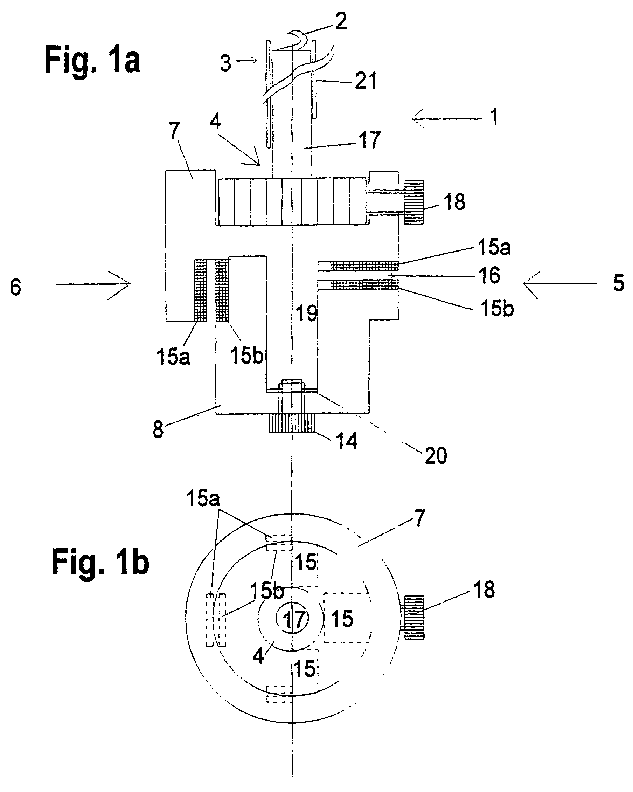

Fig. 1 zeigt den Applikator 1, bei dem der sehr lange und schmale im Schutzrohr 21 verschiebbare Applikatorstab 17 am hinteren Handhabungsende 4 eine Verdickung aufweist, mit welcher der Applikatorstab 17 in einer entsprechenden stirnseitigen Ausnehmung eines Handhabungsteiles 6, welches den Drehmomentbegrenzer 5 enthält, mittels einer Klemmschraube 18 sowohl axial fest als auch drehfest fixiert ist.

Am vorderen freien Ende, dem Sensorende 3, sitzt in dem Applikatorstab 17 der Sensor 2, der die Form einer Drahtspirale mit sehr spitzem, geschärftem Ende hat. Mit dieser Spitze soll der spiralförmige Sensor 2 z. B. in die Kopfschwarte des ungeborenen Kindes eingedreht werden.

Das Handhabungsteil 6 besteht neben dem Applikatorflansch 7, in welchem das Handhabungsende 4 des Applikatorstabes 17 aufgenommen ist, aus einem Handgriffteil 8, welches der Benutzer in der Hand hält. Der Applikatorflansch 7 weist einen nach hinten, auf der vom Applikatorstab 17 abgewandten, Stirnseite aus abragenden Fortsatz 19 mit rundem Querschnitt auf, auf welchem das Handgriffteil 8 mittels eines entsprechenden zentralen Sackloches aufgesteckt und drehend gelagert ist.

Mit Hilfe eines Vorspannungseinstellers 14 in Form einer zentralen Schraube von der Rückseite des Handgriffteiles 8 durch dieses hindurch bis in den Fortsatz 19 des Applikatorflansches 7 hinein kann die axiale Vorspannung zwischen Applikatorflansch 7 und Handgriffteil 8 zusätzlich beeinflußt werden.

In der rechten Bildhälfte der Figuren 1 stehen sich - radial außerhalb des Fortsatzes 19 - der Applikatorflansch 7 und das Handgriffteil 8 mit stirnseitigen Flächen gegenüber, wobei auf diesen gegenüberliegenden Stirnflächen Magnete 15a, 15b gegeneinander angeordnet sind. Wie Fig. 1b zeigt, sind dabei auf jeder der Stirnflächen im 90°-Abstand vier solcher Magnete 15a bzw. 15b angeordnet, und zwar so, daß sich die zwei gegeneinander gerichteten Magnete jeweils gegenseitig anziehen.

Zwischen dem Applikatorflansch 7 und dem Handgriffteil 8, die auf diese Art und Weise axial gegeneinander auf Kontakt gezogen werden, ist ein Distanzring 16 zwischengelegt. Die Dicke dieses Distanzringes 16 bestimmt bei den sich anziehenden Magnetpaarungen 15a, 15b deren gegenseitige magnetische Anziehungskraft, die sich bei Änderndem Abstand zwischen den Magneten 15a und 15b ebenfalls stark ändern würde.

Würde man nun den Applikatorflansch 7 festhalten, und das Handgriffteil 8 drehen, so ist eine bestimmte Kraft notwendig, um den, einem bestimmten Magneten 15a gerade gegenüberstehenden, Magneten 15b demgegenüber weiterzudrehen und die dazwischen bestehende magnetische Anziehungskraft zu überwinden, woraufhin sich der gedrehte Magnet 15b dem in Drehrichtung nächsten Magneten 15a gegenüber anordnen wird.

Wenn zum Einschrauben des Sensors 2 in das Gewebe des Patienten der Benutzer nicht mehr den Applikatorstab 17, sondern ausschließlich das Handgriffteil 8 des Handhabungsteiles 6 in die Hand nimmt und dreht, ist sichergestellt, daß der Sensor 2 niemals mit einem größeren Drehmoment eingeschraubt wird, als es zum Durchdrehen des Drehmomentbegrenzers 5, also zur Relativdrehung der Magnete 15b gegenüber den Magneten 15a, notwendig ist.

Benutzt man den Applikator 1 dabei zusätzlich so, daß der Benutzer angewiesen wird, am Handgriffteil 8 mindestens soweit zu drehen, bis wenigstens eine Relativdrehung zwischen den Magneten 15a und 15b stattgefunden hat, so ist weiterhin sichergestellt, daß der Sensor 2 mit einem Drehmoment, welches mindestens dem für die Überwindung der magnetischen Kraft notwendigen Drehmoment entspricht, eingedreht wurde.

In diesem Fall ist also das minimal ausgeübte Drehmoment gleich dem maximal ausgeübten Drehmoment.

In der linken Hälfte der Fig. 1 stehen sich im Gegensatz zur rechten Bildhälfte die Magnete 15a und 15b radial gegenüber. Zu diesem Zweck sind die einen Magnete 15a entlang des Außenumfanges des Handgriffteiles 8 angeordnet, und diesem Außenumfang liegt ein Innenumfang des am hinteren Ende stirnseitig hülsenförmig ausgehöhlten Applikatorflansches 7 gegenüber, in welchem mit gleicher Verteilung über den Umfang die Gegenmagnete 15a angeordnet sind.

Da wiederum das Griffteil 8 auf den Fortsatz 19 des Applikatorflansches 7 mit möglichst geringem Spiel drehend gelagert ist, nehmen die Magnete 15a und 15b immer den gleichen radialen Abstand zueinander ein, so daß auf den Einsatz einer hinsichtlich ihrer Dicke sehr wichtigen Distanzscheibe 16 wie in der rechten Hälfte der Figur 1a verzichtet werden kann, und damit auch deren Verschleiß keine Veränderung des wirkenden Drehmoments ergibt. Allerdings ist bei der in der linken Bildhälfte dargestellten Variante der Herstellungsaufwand für das Handhabungsteil 6 größer.

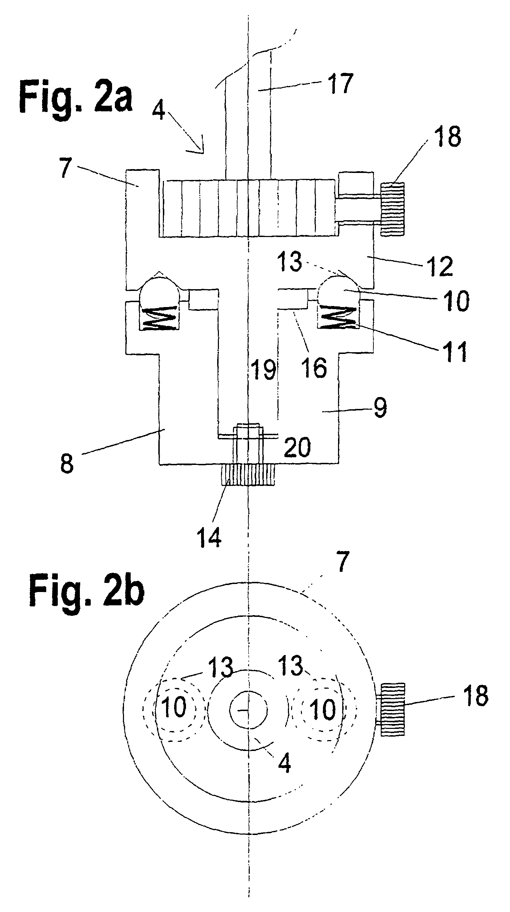

Fig. 2 zeigt demgegenüber eine mechanische Variante der Rutschkupplung, welche als Drehmomentbegrenzer 5 dient.

Wiederum besteht das Handhabungsteil 6 aus einem Flanschapplikator 7 und einem drehend demgegenüber auf dessen stirnseitigen Fortsatz 19 gelagerten Handgriffteil 8.

Dabei ist die Ausbildung des Applikatorflansches 7 sowie die Befestigung des Applikatorstabes 17 als auch die Lagerung von dem Handgriffteil 8 gegenüber dem Applikatorflansch übereinstimmend mit der Lösung gemäß Fig. 1.

Der Handgriffteil 8 dient dabei als Basisteil 9 einer Rutschkupplung, während der Applikatorflansch 7 als Gegenteil 12 der Rutschkupplung fungiert. In dem Basisteil 9 sind Rastkörper 10, beispielsweise Kugeln, in entsprechenden Vertiefungen mittels jeweils einer Feder 11 in Richtung vom Basisteil weg vorgespannt, so daß die Rastteile 10 aus dem Basisteil 9 hervorragen, in Fig. 2 dargestellt in axialer Richtung. In der gegenüberliegenden Stirnfläche des Gegenteiles 12 sind Rastvertiefungen 13 ausgebildet, in welche die Rastkörper 10 teilweise eindringen und dort formschlüssig verrasten. Den richtigen axialen Abstand zwischen dem Basisteil 9 und dem Gegenteil 12 wird wiederum durch einen axial wirkenden Vorspannungseinsteller 14, wie in Fig. 1 eine axial eingeschraubte Schraube, beibehalten, da ansonsten die Feder 11 der Rastkörper 10, Basisteil 9 und Gegenteil 12 auseinanderdrücken würde.

Radial innerhalb der Rastteile 10 ist ein Distanzring 12 zwischen dem Basisteil 9 und dem Gegenteil 12 eingelegt und mit Hilfe des Vorspannungseinstellers 14 etwas geklemmt.

Um das Basisteil und das Gegenteil 12, also den Handgriff 8 und den Flanschapplikator 7 relativ zueinander zu verdrehen, müssen die Kräfte der Federn 11 überwunden werden, was die maximale Drehmomentbegrenzung ergibt.

Wenn - wie in Fig. 2 b dargestellt - über den Umfang nur zwei solcher Verrastungen vorgesehen sind, hängt das aufzubringende Drehmoment im Drehbereich zwischen zwei Verrastungen nur von der Reibung zwischen dem Handgriffteil 8 und dem Flanschapplikator 7 ab, also der Gleitreibung gegenüber dem Distanzring 16 und der Reibung gegenüber dem Rastteil 10. Dieser Rastwiderstand stellt die minimale Drehmomentbegrenzung dar. Durch Verschrauben des Vorspannungseinstellers 14 wird der Distanzring 16 stärker zwischen den beiden benachbarten Teilen verklemmt und damit die Reibung erhöht, wodurch auch das minimal notwendige Drehmoment zum Einschrauben des Sensors 2 erhöht wird.

Die Kraft zum Überwinden der Verrastung und somit das maximal mögliche aufbringbare Drehmoment wird dadurch nicht unbedingt miterhöht, wenn die Federkennlinie der Federn 11 so gewählt wird, daß geringe axiale Annäherung von Basisteil 9 und Gegenteil 12 noch keine Erhöhung der axialen Preßkräfte auf die Rastteile 10 zur Folge hat. Selbstverständlich muß die Formschlüssigkeit zwischen dem Rastteil 10 und den Rastvertiefungen 13 so gewählt werden, daß die zur Überwindung dieser Verrastung notwendigen Kräfte grundsätzlich höher liegen als die reinen Reibungskräfte aufgrund der Reibung am Distanzring 16 und dem Rastteil 10.

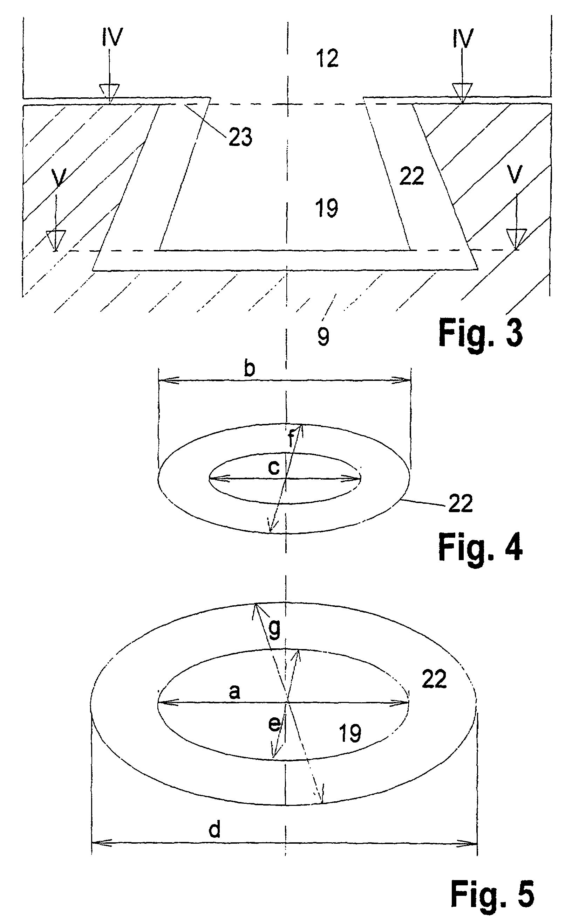

Die Figuren 3-5 zeigen eine weitere Variante einer mechanischen Rutschkupplung, die ohne bewegte Teile lediglich auf Materialelastizität beruht.

Fig. 3 zeigt einen Längsschnitt durch eine solche Rutschkupplung, welche aus einem Basisteil 9 und einem Gegenteil 12 besteht. Dabei können wiederum - wie auch anhand von Fig. 2a erläutert - Basisteil 9 und Gegenteil 12 der Applikatorflansch 7 bzw. das Handgriffteil 8 oder auch umgekehrt des Handhabungsteiles 6 bilden, welcher den Applikator 1 trägt.

Fig. 3 zeigt, daß eines des Teile, beispielsweise das Gegenteil 12, einen in axialer Richtung verlaufenden Fortsatz 19 aufweist, welcher einen sich verändernden Querschnitt, nämlich vom Ansatz am Gegenteil 12 kleinen und zum freien Ende hin kontinuierlich zunehmenden Querschnitt aufweist.

Der Querschnitt des Fortsatzes 19 ist elliptisch, und zwar mit einem großen Durchmesser a und kleinen Durchmesser e an der Stelle des größten Querschnittes, also am freien Ende des Zapfens 19, wie in der Schnittdarstellung V-V und in Fig. 5 dargestellt. Am Ansatz des Fortsatzes 19 am Gegenteil 12 ist der große Durchmesser mit c deutlich geringer als am freien Ende mit a.

Das Gegenteil 12 steckt mit diesem Fortsatz 19 in einer Ausnehmung 22 des Basisteiles 9, welches eine analoge, jedoch im Querschnitt größere Innenkontur aufweist und eine Tiefe, die etwas größer ist als die axiale Länge des Fortsatzes 19.

Da zusätzlich der größte Querschnitt des Fortsatzes 19 an dessen freien Ende noch geringfügig kleiner ist als der kleinste Querschnitt der Ausnehmung 22, welche sich an deren stirnseitiger, dem Gegenteil 12 zugewandten, Öffnung 23 befindet, kann der Fortsatz 19 in der richtigen Drehfage vollständig in die Ausnehmung 22 eingeführt werden, so daß Basisteil 9 und Gegenteil 12 stirnseitig plan aneinander anliegen. Der Querschnitt der Ausnehmung 22 ist dabei so gewählt, daß in diesem ineinandergeschobenen Zustand der kleine Durchmesser g der elliptischen Ausnehmung 22 bereits kleiner ist als der große Durchmesser a des elliptischen Zapfens 19 an der gleichen axialen Stelle.

Wenn der Querschnitt des Fortsatzes 19 sich im axialen Verlauf genauso schnell ändert wie der Querschnitt der Ausnehmung 22, also deren Flanken die gleiche Steigung gegenüber der axialen Längsrichtung aufweisen, trifft diese Bedingung auf der gesamten Länge des Fortsatzes 19 zu, und nicht nur an dessen freien Ende, wie in Fig. 5 gezeichnet.

Eine Relativdrehung um die Längsrichtung zwischen Basisteil 9 und Gegenteil 12 um 90° oder mehr ist dabei nur möglich, wenn durch Materialelastizität sich entweder der große Durchmesser des Fortsatzes 19 und/oder der kleine Durchmesser der umgebenden Innenkontur der Ausnehmung 22 so verformt, daß ein Durchrutschen der beiden Teile gegeneinander möglich ist.

Somit bestimmt neben der Materialelastizität des Fortsatzes 19 des Gegenteiles 12 bzw. des Basisteiles 9 auch das Übermaß zwischen dem großen Durchmesser des Fortsatzes 19 und dem an der gleichen Stelle vorhandenen kleinen Durchmesser des elliptischen Querschnittes der Ausnehmung 22 die Kraft, welche zum Durchdrehen dieser formschlüssigen mechanischen Rutschkupplung notwendig ist.

## Patentansprüche

1. Applikator zum Eindrehen eines Sensors in die Haut eines Lebewesens, wobei der Applikator (1) mit einem Drehmomentbegrenzer (5) wirkverbunden ist.
**dadurch gekennzeichnet, daß**
der Drehmomentbegrenzer (5) so ausgebildet ist,
(a) daß er eine Wahrnehmung des Durchrutschens am Schwellenwert des Drehmomentbegrenzers (5) ermöglicht,
(b) daß der Schwellenwert des Drehmomentbegrenzers (5) so gewählt ist, daß der Applikator (1) das Eindrehen mit mindestens einem minimalen, zur sicheren Verankerung des Sensors in der Haut notwendigen Drehmoment und höchstens mit einem maximalen, noch sicheren Drehmoment, gewährleistet, so daß Verletzungen des Lebewesens weitgehend vermieden werden und eine uneingeschränkte Funktion des Sensors gewährleistet ist.

2. Applikator nach Anspruch 1,
**dadurch gekennzeichnet, daß**
eine Wahrnehmung mittels der den Applikator handhabenden Hand des Benutzers möglich ist.

3. Applikator nach einem der vorhergehenden Ansprüche,
**dadurch gekennzeichnet, daß**
der Applikator (1) länglich stabförmig ausgebildet ist mit einem vorderen, den Sensor (2) lösbar tragenden Sensorende (3) und einem hinteren Handhabungsende (4).

4. Applikator nach einem der vorhergehenden Ansprüche,
**dadurch gekennzeichnet, daß**
der Applikator (1) als Einwegteil konzipiert ist.

5. Applikator nach einem der vorhergehenden Ansprüche,
**dadurch gekennzeichnet, daß**
der Drehmomentbegrenzer (5) zwischen dem Applikator (1) und dem Sensor (2) am Sensorende (3) angeordnet ist.

6. Applikator nach einem der Ansprüche 1 bis 3,
**dadurch gekennzeichnet, daß**
der Drehmomentbegrenzer (5) im Bereich des Handhabungsendes (4) angeordnet ist.

7. Applikator nach Anspruch 6,
**dadurch gekennzeichnet, daß**
ein Handhabungsteil (6) lösbar am Handhabungsende (4) des Applikators (1) angeordnet ist und das Handhabungsteil (6) wiederverwendbar ist.

8. Applikator nach Anspruch 7,
**dadurch gekennzeichnet, daß**
der Drehmomentbegrenzer (5) zwischen dem Handhabungsende (4) des Applikators (1) und dem Handhabungsteil (6) angeordnet ist.

9. Applikator nach Anspruch 6 oder 7,
**dadurch gekennzeichnet, daß**
der Drehmomentbegrenzer (5) im Handhabungsteil (6) zwischen einem Applikatorflansch (7) und einem Handgriffteil (8), welche Bestandteil des Handhabungsteils (6) sind, angeordnet sind.

10. Applikator nach Anspruch 9,
**dadurch gekennzeichnet, daß**
der Applikatorflansch (7) mit dem Handgriffteil (8) drehbar aber in axialer Richtung fest miteinander verbunden sind.

11. Applikator nach Anspruch 8,
**dadurch gekennzeichnet, daß**
der Drehmomentbegrenzer (5) eine Rutschkupplung, insbesondere eine mechanische, kraft- oder formschlüssige, Rutschkupplung ist.

12. Applikator nach Anspruch 11,
**dadurch gekennzeichnet, daß**
die Rutschkupplung eine magnetische, insbesondere eine elektromagnetische Rutschkupplung ist.

13. Applikator nach einem der vorhergehenden Ansprüche,
**dadurch gekennzeichnet, daß**
der Drehmomentbegrenzer (5) eine Vorrichtung zum Wahrnehmen, insbesondere Wahrnehmen mittels der handhabenden Hand, des Durchrutschens des Drehmomentbegrenzers umfaßt.

14. Applikator nach Anspruch 12,
**dadurch gekennzeichnet, daß**
die magnetische Rutschkupplung aus einem Basisteil (9) und einem demgegenüber drehbar befestigten Gegenteil (12) besteht, und in den zwei gegeneinander gerichteten Flächen des Basisteiles (9) und des Gegenteiles (12), vorzugsweise an zwei gegeneinander gerichteten Stirnflächen, gegeneinander gerichtete Magnete (15a, 15b) angeordnet sind.

15. Applikator nach Anspruch 11,
**dadurch gekennzeichnet, daß**
die mechanische Rutschkupplung ein Basisteil (9) und ein demgegenüber drehbar befestigtes Gegenteil (12) umfaßt, wobei aus einem der Teile Rastteile (10) vorstehen, die in Richtung ihres Vorstehens vorgespannt sind, und in dem anderen Teil Rastvertiefungen (13), in die die Rastteile (10) einrasten können.

16. Applikator nach Anspruch 14,
**dadurch gekennzeichnet, daß**
an dem Basisteil (9) die Magnete (15a) alle mit dem gleichen Pol gegen das Gegenteil (12) ausgerichtet angeordnet sind und am Gegenteil (12) die Magnete (15b) alle mit dem anderen Pol gegen das Basisteil (9) ausgerichtet angeordnet sind.

17. Applikator nach Anspruch 14,
**dadurch gekennzeichnet, daß**
bei Anordnung der Magnete (15a, b) in axialer Richtung zwischen dem Basisteil (9) und dem Gegenteil (12) ein Distanzring (16) aus nicht magnetisierbarem Material angeordnet ist, der eine möglichst geringe, insbesondere keine, Haftreibung und/oder Losbrecheffekt beim Lösen der Haftung aufweist.

18. Applikator nach einem der vorhergehenden Ansprüche 14 bis 17,
**dadurch gekennzeichnet, daß**
die Vorspannung in axialer Richtung zwischen Basisteil (9) und Gegenteil (12) mittels eines Vorspannungseinstellers (14) einstellbar ist.

19. Applikator nach einem der vorhergehenden Ansprüche 14, 16 oder 17,
**dadurch gekennzeichnet, daß**
die Magnete (15a, b) in radial konzentrisch zueinander angeordneten Ringflächen des Basisteiles (9) und des Gegenteiles (12) angeordnet sind.

20. Applikator nach Anspruch 11,
**dadurch gekennzeichnet, daß**
die Rutschkupplung eine mechanische Kupplung ist mit einem Basisteil (9) und einem demgegenüber drehbar gelagerten Gegenteil (12), wobei eines der Teile einen Fortsatz (9) mit unrunder, insbesondere elliptischer oder vieleckiger Außenkontur aufweist und in einer Ausnehmung (22) mit insbesondere analog geformter vieleckiger Innenkontur, von der es wenigstens teilweise umschlossen wird, drehbar gelagert ist und das Basisteil (9) und/oder das Gegenteil (12) eine so große Materialelastizität aufweisen, daß durch Verformung eines der unrunden Konturen eine Relativdrehung zwischen Basisteil und Gegenteil möglich ist.

21. Applikator nach Anspruch 20,
**dadurch gekennzeichnet, daß**
das Gegenteil (12) einen Fortsatz (19) mit elliptischem Querschnitt aufweist, dessen Querschnitt sich vom Gegenteil (12) zum freien Ende hin vergrößert, insbesondere konisch vergrößert, und das Basisteil (9) eine analog geformte größere Ausnehmung (22) mit analoger Innenkontur aufweist, wobei
- der größte Querschnitt des Fortsatzes (19) des Gegenteiles (12) geringfügig kleiner ist als der kleinste Querschnitt der Ausnehmung (22), und
- bei vollständig in der Ausnehmung (22) des Basisteiles (9) eingestecktem Fortsatz (19) des Gegenteiles (12) an wenigstens einer Stelle der axialen Erstreckung des Fortsatzes (19) der große Durchmesser (a) des Querschnittes des Fortsatzes (19) größer ist als der kleine Durchmesser (g) des Querschnittes der Ausnehmung (22) an dieser axialen Stelle.

22. Applikator nach Anspruch 21,
**dadurch gekennzeichnet, daß**
der Querschnitt des Fortsatzes (19) und der Ausnehmung (22) in axialer Richtung, also vom Ansatz des Fortsatzes (19) am Gegenteil (12) zu dessen freien Ende hin, kontinuierlich zunehmen.

23. Applikator nach einem der vorhergehenden Ansprüche 21 oder 22,
**dadurch gekennzeichnet, daß**
der Fortsatz (19) in axialer Richtung kürzer ist als die Tiefe der Ausnehmung (22).

24. Applikator nach einem der vorhergehenden Ansprüche, 14 bis 23 und nach Anspruch 9
**dadurch gekennzeichnet, daß**
der Applikatorflansch (7) und das Handgriffteil (8) des Handhabungsteiles (6) das Basisteil (9) bzw. Gegenteil (12) der Rutschkupplung (5) bilden.

25. Applikator nach Anspruch 24,
**dadurch gekennzeichnet, daß**
der Außenumfang des Handgriffteiles (8) etwa den gleichen Durchmesser besitzt wie der Außendurchmesser des hinteren, direkt zu greifenden Endes eines herkömmlichen Einweg-Applikators ohne Drehmomentbegrenzer.

## Claims

1. Applicator for screwing a sensor into the skin of a living being, whereby the applicator (1) is coupled with the torque limiter (5).
**thereby characterized, that**
the torque limiter (5) is designed in a manner
(a) that the torque limiter (5) allows a perception feedback of the slipping-through phenomenon at the threshold value of the torque limiter
(b) that the threshold value of the torque limiter (5) is adjusted so, that the applicator (1) ensures the application of the sensor by turning procedure to screw in the sensor with at least a minimal torque necessary to securely anchor the sensor in the skin and, on the other hand, with a maximal, but still save torque, so that injuries can be avoided considerably and a unlimited use of the sensor is ensured.

2. Applicator according to claim 1,
**thereby characterized, that**
a perception feedback to the operators hand while handling the applicator becomes possible

3. Applicator according to one of the preceding claims,
**thereby characterized, that**
the applicator (1) is basically rod-shaped, and further adapted for a releasable seating of the sensor (2) at a distal sensor end, and adapted for manipulation by hand at a proximal handling end (4)

4. Applicator according to one of the preceding claims,
**thereby characterized, that**
the applicator (1) is designed as a single use item.

5. Applicator according to one of the preceding claims,
**thereby characterized, that**
the torque limiter (5) is positioned between the applicator (1) and the sensor (2) near to the sensor's end (3).

6. Applicator according to claim 1 to 3,
**thereby characterized, that**
the torque limiter (5) is positioned near to the applicators handling end (4)

7. Applicator according to claim 6,
**thereby characterized, that**
a manipulation part (6) is positioned removably at the handling end (4) of the applicator (1) and wherein the manipulation part (6) is reusable.

8. Applicator according to claim 7,
**thereby characterized, that**
the torque limiter (5) is positioned between the handling end (4) of the applicator (1) and the manipulation part (6).

9. Applicator according to claim 6 or 7,
**thereby characterized, that**
the torque limiter (5) is positioned in the manipulation part (6) between the applicator flange (7) and the grip part (8), which are both components of the manipulation part (6).

10. Applicator according to claim 9,
**thereby characterized, that**
the applicator flange (7) is rotatably connected with the hand grip part (8) but fixed in the axial direction with respect to the hand grip part (8).

11. Applicator according to claim 8,
**thereby characterized, that**
the torque limiter (5) is a mechanical slip clutch, in particular a force slip clutch or a form locking slip clutch.

12. Applicator according to claim 11,
**thereby characterized, that**
the slip clutch is a magnetic one, in particular an electro-magnetic slip clutch.

13. Applicator according to one of the preceding claims,
**thereby characterized, that**
the torque limiter (5) is a device to perceive the slipping through phenomenon of the torque limiter, in particular to perceive with the manipulating hand.

14. Applicator according to claim 12,
**thereby characterized, that**
the magnetic slip clutch comprising a base part (9) and an opposing counterpart (12) rotatably secured thereto, wherein at least two opposing set of magnets (15a, 15b) are directed against each other in two opposing faces of the base part (9) and the counterpart (12).

15. Applicator according to claim 11,
**thereby characterized, that**
the mechanical slip clutch comprises of a base part (9) and an opposing rotatably mounted counterpart (12), wherein engagement parts (10) project from either one of the base part (9) or the counterpart (12), and detent recesses (13) are formed in the respective part (9), the engagement parts (10) which in the direction of its projection are pre-tensioned, and configured to engage in detent recesses (13) formed in the opposing part.

16. Applicator according to claim 14,
**thereby characterized, that**
in the base part (9) all magnets (15a) are oriented in the same polarity against the counterpart (12) and all magnets (15b) of the counterpart (12) are oriented in the opposite polarity against the magnets 15a of the base part (9).

17. Applicator according to claim 14,
**thereby characterized, that**
in arranging the magnets a distance ring (16) of non-magnetic material is positioned in the axial direction between the base part (9) and the counterpart (12), the distance ring (16) comprising a minimal or no static friction and / or no sudden friction release effect when the friction is overcome between the base part (9) and the counterpart (12).

18. Applicator according to one of the preceding claims 14 - 17,
**thereby characterized, that**
the pre-tension in the axial direction between the base part (9) and the counterpart (12) is adjustable by means of a pre-tension adjusting mechanism (14).

19. Applicator according to one of the preceding claims 14,16 or 17,
**thereby characterized, that**
the magnets (15a,b) are assembled so that radial concentric ring surfaces in the base part (9) and in the counterpart (12) are facing each other.

20. Applicator according to claim 11,
**thereby characterized, that**
the slip clutch is designed as a mechanical clutch comprising a base part (9) and a counterpart (12) wherein one of the parts comprises a protrusion (19) with a non-round, preferably elliptic or polygonal outer contour, and is rotatably mounted thereto in a recess (22) having an inner contour which is predominately the analogous shape of the nonround, elliptic or polygonal inner contour of said protrusion and which at least partially surrounds it, and the base part (9) and / or the counterpart (12) is made of a material possessing a material elasticity adequate to allow a relative motion between the base part (9) and the counter part (12) by producing a deformation of one of the non-round contours.

21. Applicator according to claim 20,
**thereby characterized, that**
the counterpart (12) exhibits a protrusion (19) with elliptical cross-section, of which the cross-section dimensions increase towards the free end, and the base part (9) exhibits an analogously shaped larger recess (22) with analogous inner contour, wherein the largest cross-section of the protrusion (19) of the counterpart (12) is slightly smaller than the smallest cross-section of the recess (22) of the base part (9);
- the largest cross-section of the protrusion (19) of the counterpart (12) is slightly smaller than the smallest cross-section of the recess (22), and
- at least at one little spot of the axial length of the protrusion (19) the long diameter (a) of the protrusions' cross section (19) is larger than the small diameter of the recesses cross section, if the protrusion (19) is fully inserted into the recess.

22. Applicator according to claim 21,
**thereby characterized, that**
the cross-section of the protrusion (19) and the cross-section of the recess (22) continuously increase in the axial direction from the base of the protrusion (19) at the counterpart (12) towards the free end thereof.

23. Applicator according to one of the preceding claims 21 or 22,
**thereby characterized, that**
in the axial direction the protrusion (19) is shorter than the depth of the recess (22).

24. Applicator according to one of the preceding claims 14 - 23 and claim 9,
**thereby characterized, that**
the slip clutch (5) comprises means for limiting torque transfer between the applicator flange (7) and the hand grip part (8) of the manipulation part (6) of the base part (9).

25. Applicator according to claim 24,
**thereby characterized, that**
the outer diameter of the grip part (8) has about the same diameter as the outer diameter of the proximal direct handling end of a common one way applicator without a torque limiter being attached.

## Revendications

1. Applicateur permettant d'introduire un détecteur dans la peau d'un être vivant, l'action de l'applicateur (1) était soumise à un limiteur de couple (5),
**caractérisé par le fait que**
le limiteur de couple (5) est constitué de sorte
(a) qu'il permet de percevoir le glissement du limiteur de couple (5) lorsque la valeur seuil est atteinte,
(b) que la valeur seuil du limiteur de couple (5) est choisie de manière à ce que l'applicateur (1) assure une pénétration avec au moins un couple minimum nécessaire pour un ancrage sûr du détecteur dans la peau, et au plus un couple maximum encore sûr, de sorte que toute blessure de l'être vivant soit en grande partie évitée et qu'un fonctionnement illimité du détecteur soit garanti.

2. Applicateur selon la revendication 1
**caractérisé par le fait que**
le glissement du limiteur de couple (5) peut être ressenti.

3. Applicateur selon la revendication 1
**caractérisé par le fait que**
l'applicateur (1) se présente sous la forme d'une baguette oblongue, composée d'une extrémité de détecteur avant (3) pouvant être détachée du détecteur (2) et d'une extrémité arrière servant à la manipulation (4).

4. Applicateur selon une des revendications précitées
**caractérisé par le fait que**
l'applicateur (1) est un élément jetable.

5. Applicateur selon une des revendications précitées
**caractérisé par le fait que**
le limiteur de couple (5) est situé entre l'applicateur (1) et le détecteur (2) à l'extrémité du détecteur.

6. Applicateur selon une des revendications 1 à 3
**caractérisé par le fait que**
le limiteur de couple (5) est situé dans la zone de l'extrémité de manipulation (4).

7. Applicateur selon la revendication 6
**caractérisé par le fait que**
un élément de manipulation (6) est monté de manière amovible sur l'extrémité de manipulation (4) de l'applicateur (1) et que l'élément de manipulation (6) peut être réutilisé.

8. Applicateur selon la revendication 7
**caractérisé par le fait que**
le limiteur de couple (5) est situé entre l'extrémité de manipulation (4) de l'applicateur (1) et l'élément de manipulation (6).

9. Applicateur selon la revendication 6 ou 7
**caractérisé par le fait que**
le limiteur de couple (5) est disposé dans l'élément de manipulation (6), entre une bride d'applicateur (7) et une pièce de poignée (8), qui forment les éléments de l'élément de manipulation (6).

10. Applicateur selon la revendication 9
**caractérisé par le fait que**
la bride de l'applicateur (7) est reliée à la pièce de poignée (8) de manière à pouvoir toumer, mais fixe sur le plan axial.

11. Applicateur selon la revendication 8
**caractérisé par le fait que**
le limiteur de couple (5) est un accouplement patinant, et en particulier un accouplement patinant à crabot ou entraîné par adhérence.

12. Applicateur selon la revendication 11
**caractérisé par le fait que**
l'accouplement patinant est un accouplement patinant magnétique, et particulièrement électromagnétique.

13. Applicateur selon une des revendications précitées
**caractérisé par le fait que**
le limiteur de couple (5) possède un dispositif permettant de percevoir, particulièrement avec la main traitante, le glissement du limiteur de couple.

14. Applicateur selon la revendication 12
**caractérisé par le fait que**
l'accouplement patinant magnétique se compose d'une pièce de base (9) et d'une contre pièce pivotante disposées face à face (12) et que des aimants (15a, 15b) sont montés de manière opposée dans les deux surfaces opposées de la pièce de base (9) et de la contre pièce (12), de préférence sur les deux surfaces extérieures opposées.

15. Applicateur selon la revendication 11
**caractérisé par le fait que**
l'accouplement patinant mécanique se compose d'une pièce de base (9) et d'une contre pièce (12), ainsi que d'une partie pivotante fixée sur le côté opposé, des pièces d'arrêt (10) dépassant d'une des pièces prétendues dans le sens de la saillie et d'encoches (13) sur l'autre pièce, dans lesquelles les pièces d'arrêt (10) peuvent s'enclencher.

16. Applicateur selon la revendication 14
**caractérisé par le fait que**
les aimants (15a) sont tous disposés sur la pièce de base (9), le même pôle dirigé vers la contre pièce (12) et que les aimants (15b) sont montés sur la contre pièce (12), l'autre pôle dirigé vers la pièce de base (9).

17. Applicateur selon la revendication 14
**caractérisé par le fait que**
lorsque les aimants (15a) sont montés dans le sens axial, une rondelle d'écartement (16) en matériau non magnétique est introduite entre la pièce de base (9) et la contre pièce (12), opposant un frottement par adhérence aussi faible que possible, au mieux inexistant, et/ou un effet de décollement lorsque l'adhérence est coupée.

18. Applicateur selon une des revendications précédentes 14 à 17
**caractérisé par le fait que**
la prétension dans le sens axial est réglable entre la pièce de base (9) et la contre pièce (12) grâce à un dispositif de réglage de la prétension (14).

19. Applicateur selon une des revendications précédentes 14,16 ou 17
**caractérisé par le fait que**
les aimants (15a, b) sont montés dans des surfaces annulaires radiales concentriques les unes par rapport aux autres de la pièce de base (9) et de la contre pièce (12).

20. Applicateur selon la revendication 11
**caractérisé par le fait que**
l'accouplement patinant est un accouplement mécanique composé d'une pièce de base (9)
et d'une contre pièce pivotante (12), une des pièces étant un prolongement (9) présentant un contour extérieur en faux rond, elliptique ou carré, et montée de manière pivotante dans un creux (22) présentant un contour de forme carrée, par lequel elle est au moins partiellement enveloppée, et que la pièce de base (9) et/ou la pièce opposée (12) présente une élasticité de matériau telle qu'un pivotement relatif entre la pièce de base et la contre pièce est rendu possible par la déformation d'un des contours elliptiques.

21. Applicateur selon la revendication 20
**caractérisé par le fait que**
la contre pièce (12) est un prolongement (19) présentant une section elliptique dont la section augmente de la contre pièce (12) à son extrémité, et en particulier une augmentation conique, et que la pièce de base (9) présente un creux (22) de forme analogue, avec un contour intérieur analogue, considérant que
- la section la plus importante du prolongement (19) de la contre pièce (12) est légèrement plus faible que la section la plus faible du creux (22) et que
- lorsque le prolongement (19) de la pièce opposée (12) est entièrement introduit dans le creux (22) de la pièce de base (9), le grand diamètre (a) de la section du prolongement (19) est plus important, au moins à un endroit de la distance axiale du prolongement (19) que le petit diamètre (g) du creux (22) sur ce côté axial.

22. Applicateur selon revendication 21
**caractérisé par le fait que**
la section du prolongement (19) et du creux (22) augmentent sans cesse dans le sens axial, donc du départ du prolongement (19) sur la contre pièce (12) à son extrémité.

23. Applicateur selon une des revendications précédentes 21 ou 22
**caractérisé par le fait que**
le prolongement (19) est plus court dans le sens axial que la profondeur du creux (22).

24. Applicateur selon une des revendications précédentes 14 à 23, revendication 9
**caractérisé par le fait que**
la bride de l'applicateur (7) et la pièce de poignée (8) de l'élément de manipulation (6) forment la pièce de base (9) ou la contre pièce (12) de l'accouplement patinant.

25. Applicateur selon revendication 24
**caractérisé par le fait que**
la circonférence extérieure de la pièce de poignée (8) présente à peu près le même diamètre que le diamètre extérieur de l'extrérnité arrière à saisir d'un applicateur traditionnel jetable sans limiteur de couple.
